# EUROPÄISCHE PATENTSCHRIFT

(11) **EP 0 848 603 B1**
(45) Veröffentlichungstag und Bekanntmachung des Hinweises auf die Patenterteilung: **20.03.2002**
(21) Anmeldenummer: 97925873.8
(22) Anmeldetag: 22.05.1997
(51) Int. Cl.: A61F 2/44

(54) **IMPLANTAT ZUM EINSETZEN ZWISCHEN WIRBELKÖRPER ALS PLATZHALTER**
IMPLANT FOR FILLING A SPACE BETWEEN VERTEBRAE
IMPLANT A INSERER ENTRE DES VERTEBRES POUR OCCUPER UN ESPACE VIDE

(30) Priorität: 07.06.1996 DE 19622827
(43) Veröffentlichungstag der Anmeldung: 24.06.1998
(73) Patentinhaber: ULRICH, Heinrich, 89075 Ulm/Donau (DE)
(72) Erfinder: SCHÖNHÖFFER, Helmut, D-89077 Ulm (DE)
(74) Vertreter: Dziewior, Joachim, Dipl.-Phys. Dr.
(86) Internationale Anmeldenummer: DE9701065
(87) Internationale Veröffentlichungsnummer: WO9747258

(56) Entgegenhaltungen:
- DE-A- 4 409 392
- DE-A- 4 423 257
- DE-A- 19 509 317
- DE-C- 4 323 956
- FR-A- 2 666 221
- FR-A- 2 730 158
- US-A- 4 502 160
- US-A- 5 358 524

## Beschreibung

Die Erfindung betrifft ein Implantat zum Einsetzen zwischen Wirbelkörper als Platzhalter, bestehend aus zwei endständigen, zur Anlage an den angrenzenden Wirbeln bestimmten Implantatteilen und einem dazwischen befindlichen mittleren Implantatteil, das mit einem der endständigen Inplantatteile durch ein Gewinde verbunden ist, wobei die drei Implantatteile und das Gewinde in Längsrichtung der Wirbelsäule koaxial angeordnet sind und durch Verdrehen des mittleren Implantatteils die Länge des Implantats insgesamt veränderbar ist, und wobei die endständigen Implantatteile rohrförmige Hülsen mit von Aussparungen durchbrochenen Hülsenwänden bilden.

Implantate dieser Art sind aus DE 44 23 257 A1 bekannt und dienen als Platzhalter für aus der Wirbelsäule entfernte Wirbel oder Wirbelteile. Die Implantate ermöglichen durch Verdrehen des mittleren Implantatteils eine Distraktion des das Implantat enthaltenden Wirbelsäulenbereichs. Dabei sind beide endständigen Implantatteile mit dem mittleren Implantatteil durch je ein eigenes Gewinde verbunden, wobei die beiden Gewinde in Längsrichtung der Wirbelsäule koaxial angeordnet sind und zueinander gegenläufigen Gewindesinn besitzen. Durch die Aussparungen in den Implantatteilen kann gewünschtenfalls ein in das Innere der rohrförmigen Hülsen einzubringendes Material, wie Knochenzement oder Knochenstücke, eingefügt werden, wodurch ein schnellerer Gefäßanschluß mit dem Implantat erreicht wird. Daher ist das Implantat für die Induktion der Knochenbildung und Stimulation der Knochen gut geeignet und es kann nach der Implantation leicht und schnell einwachsen. Allerdings ist bei diesen Implantaten die axiale Gesamtlänge mindestens so groß wie die Gesamtlänge der beiden gegensinnigen Gewinde, was eine Beschränkung hinsichtlich der kleinstmöglichen Implantatlänge bedeutet, so daß diese bekannten Implantate nicht als Zwischenwirbelplatzhalter zum Bandscheibenersatz geeignet sind.

Der Erfindung liegt die Aufgabe zu Grunde, ein Implantat der eingangs genannten Art so auszubilden, daß es auf eine Mindestlänge einstellbar ist, die seine Verwendung außer als kompletter Ersatz eines Wirbelkörpers, insbesondere im Halswirbelbereich, auch als Zwischenwirbelplatzhalter zum Bandscheibenersatz, insbesondere im lumbalen Bereich, ermöglicht.

Diese Aufgabe wird nach der Erfindung dadurch gelöst, daß die beiden endständigen Implantatteile mit ihren Hülsen axial ineinander greifen und an den Hülsenwänden axial gegeneinander verschieblich geführt sowie gegen gegenseitiges Verdrehen um die Hülsenachse gesichert sind, daß das Gewinde zwischen dem mittleren Implantatteil und dem einzigen, mit ihm gewindemäßig verbundenen endständigen Implantatteil an dessen Hülse vorgesehen ist und daß das im Gewinde dieser Hülse verdrehbare mittlere Implantatteil als axialer Anschlag für das andere endständige Implantatteil ausgebildet ist.

Da das erfindungsgemäße Implantat nur ein einziges Gewinde besitzt, ist die mögliche Mindestlänge, auf die das Implantat verkürzt werden kann, durch die Gewindelänge nur dieses einen Gewindes bestimmt, denn die beiden endständigen Implantatteile können praktisch über die gesamte Länge ihrer rohrförmigen Hülsen völlig ineinandergeschraubt werden, was insgesamt eine axial sehr niedrige Bauform und damit die Verwendung des Implantats als Bandscheibenersatz ermöglicht. Dennoch bleibt die Distraktionsmöglichkeit des Implantats während der Operation voll erhalten, da nur das mittlere Implantatteil im Gewinde entsprechend verdreht zu werden braucht, damit sich die Länge des Implantats insgesamt nach Wunsch ändert.

Eine im Hinblick auf die Herstellung, Montage und Handhabung besonders einfache und daher bevorzugte Ausführungsform des erfindungsgemäßen Implantats ist dadurch gekennzeichnet, daß die Hülse des mit dem mittleren Implantatteil gewindemäßig verbundenen endständigen Implantatteils das Gewinde auf seiner äußeren Umfangsfläche trägt und sowohl vom mittleren Implantatteil als auch von der Hülse des anderen endständigen Implantatteils radial außen umfaßt ist. Dadurch ist das mittlere Implantatteil zum Verdrehen leicht zugänglich und das dieses mittlere Implantatteil führende Gewinde des endständigen Implantatteils durch die Hülse des anderen endständigen Implantatteils außenseitig axial übergriffen. Auch empfiehlt es sich, die Anordnung so zu treffen, daß zur gegenseitigen Verdrehungssicherung der beiden endständigen Inplantatteile in der Hülsenwand des einen Inplantateils ein axial verlaufender Führungsschlitz und an der Hülsenwand des anderen Inplantatteils ein in den Führungsschlitz eingreifender Führungsvorsprung vorgesehen ist. Führungsschlitz und Führungsvorsprung können zu mehreren verteilt über den Umfang der Hülsenwände angeordnet sein. Um die gegenseitige Stellung der beiden endständigen Implantatteile auch unabhängig von der Anschlagfunktion des mittleren Implantatteils fixieren zu können, ist eine weitere vorteilhafte Ausführungsform des erfindungsgemäßen Implantats dadurch gekennzeichnet, daß die Hülse eines der endständigen Implantatteile im Überlappungsbereich der Hülsen beider endständiger Implantatteile mindestens eine radiale Gewindebohrung aufweist, in der eine gegen die Hülse des anderen endständigen Implantatteils verspannbare Klemmschraube geführt ist. In weiter bevorzugter Ausführungsform können eines oder beide der endständigen Implantatteile am jeweils vom mittleren Implantatteil abgewandten Ende mit einer ringförmigen Stirnplatte und diese Stirnplatte an ihrer Stirnfläche mit zum Eindringen in den angrenzenden Wirbel bestimmten Schneiden oder Spitzen versehen sein. Bezüglich der Ausbildung und Anordnung der Aussparungen in den Hülsenwänden der endständigen Implantatteile besteht im Rahmen der Erfindung eine gewisse Wahlfreiheit. Einerseits soll der von den Aussparungen insgesamt repräsentierte lichte Querschnitt möglichst groß sein, anderseits dürfen die Aussparungen den Gewindebereich nicht unzulässig schwächen, damit die Festigkeit der Gewindeverbindung zwischen dem einen endständigen Implantatteil und dem damit gewindemäßig verbundenen mittleren Implantatteil keine Beeinträchtigung erfährt. Im allgemeinen wird man dabei die Aussparungen als im Querschnitt kreisförmige Bohrungen oder als Langlöcher ausbilden und gleichmäßig über den Hülsenumfang verteilt anordnen. Weitere Aussparungen sind vorzugsweise in der äußeren Umfangsfläche des mittleren Implantatteils über den Umfang verteilt angeordnet und als Schlüsselöffnungen zum Einstecken eines zum Verdrehen des mittleren Implantatteiles dienenden Schlüssels ausgebildet.

Im folgenden ist die Erfindung an einem in der Zeichnung dargestellten Ausführungsbeispiel näher erläutert; es zeigen:
- Fig. 1: Ein Implantat nach der Erfindung in einer Seitenansicht und in dem Zustand, in dem es auf seine kleinstmöglich axiale Länge eingestellt ist,
- Fig. 2: den Gegenstand der Fig. 1, jedoch im Zustand, in dem das Implantat auf seine größtmögliche Länge eingestellt ist,

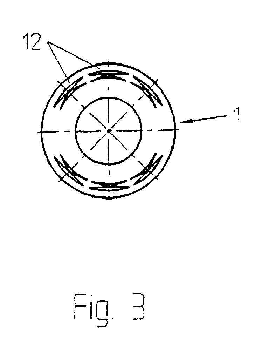

- Fig. 3: eine Stirnansicht des Implantats,

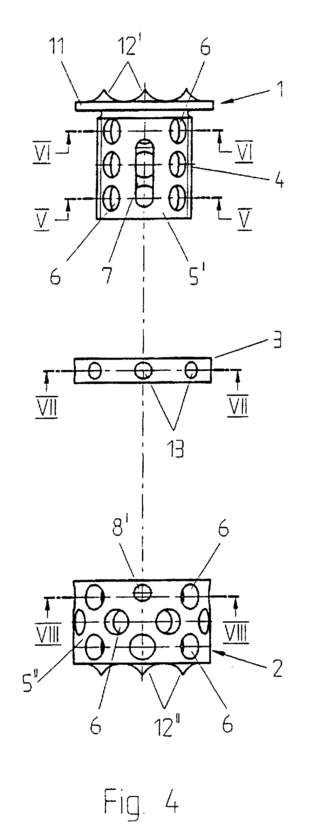

- Fig. 4: das Implantat nach den Fig.1 und 2 in axial auseinandergezogenem Zustand der Implantatteile,

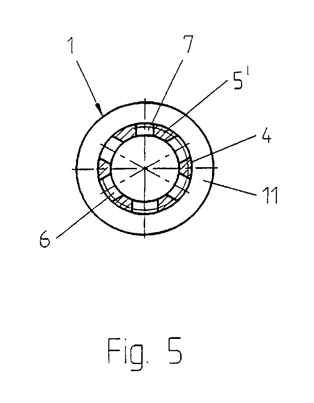

- Fig. 5: den Schnitt V - V in Fig. 4,

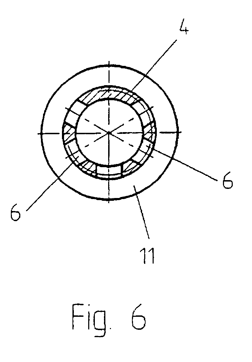

- Fig. 6: den Schnitt VI - VI in Fig. 4,

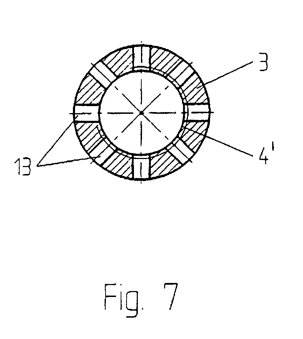

- Fig. 7: den Schnitt VII - VII in Fig. 4 und

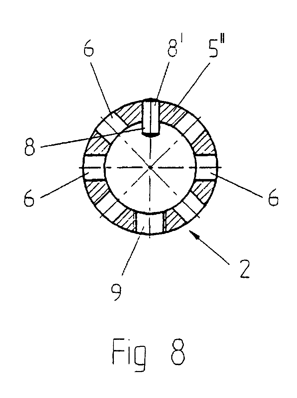

- Fig. 8: den Schnitt VIII - VIII in Fig. 4.

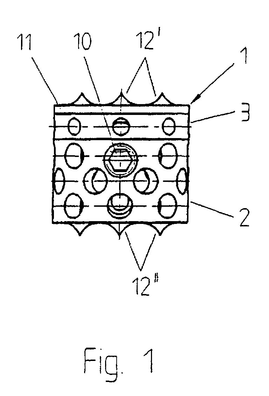

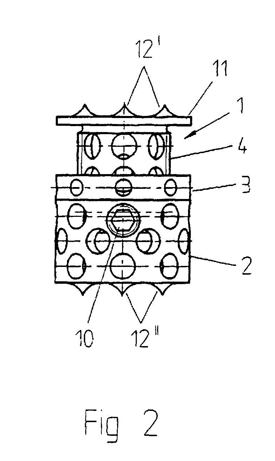

Das in der Zeichnung dargestellte Implantat dient zum Einsetzen zwischen in der Zeichnung selbst nicht dargestellte Wirbel als Platzhalter, und zwar, je nach Längeneinstellung des Implantats, zum Bandscheibenersatz oder für aus der Wirbelsäule entfernte Wirbel oder Wirbelteile. Das Implantat besteht aus zwei endständigen, zur Anlage an den jeweils angrenzenden Wirbeln bestimmten Implantteilen 1, 2 und einem dazwischen befindlichen mittleren Implantatteil 3. Das mittlere Implantatteil 3 ist mit einem der beiden endständigen Implantatteile, in der Zeichnung jeweils mit dem oberen endständigen Implantatteil 1, durch ein Gewinde 4 verbunden. Die drei Implantatteile 1 bis 3 und das Gewinde 4 sind in Längsrichtung der Wirbelsäule koaxial angeordnet. Durch Verdrehen des mittleren Implantatteils 3 im Gewinde 4 ist die Länge des Implantats insgesamt zwischen den aus dem Vergleich der Fig. 1 und 2 ersichtlichen Grenzen veränderbar. Die endständigen Implantatteile 1, 2 bilden rohrförmige Hülsen 5', 5" mit von Aussparungen 6 durchbrochenen Hülsenwänden, wobei diese Aussparungen 6 ein schnelles Einwachsen des Implantats im umgebenden Gewebe ermöglichen. Die beiden endständigen Implantatteile 1, 2 greifen mit ihren Hülsen 5', 5" axial ineinander. Sie sind an den Hülsenwänden axial gegeneinander verschieblich geführt sowie gegen gegenseitiges Verdrehen um die Hülsenachse in noch zu erläuternder Weise gesichert. Das Gewinde 4 zwischen dem mittleren Implantatteil 3 und dem mit ihm gewindemäßig verbundenen endständigen, in der Zeichnung also oberen Implantatteil 1 ist an dessen Hülse 5' vorgesehen. Das im Gewinde 4 dieser Hülse 5' verdrehbare mittlere Implantatteil 3 ist als axialer Anschlag für die Hülse 5" des anderen, in der Zeichnung unteren endständigen Implantatteils 2 ausgebildet. Durch Verdrehen des mittleren Implantatteils 3 im Gewinde 4 des oberen endständigen Implantatteils 1 ändert sich die axiale Lage des mittleren Implantatteils 3 auf dem oberen endständigen Implantatteil 1, so daß sich auch die axiale Lage der beiden endständigen Implantatteile 1 zueinander ändert, da die Lage des in der Zeichung unteren endständigen Implantatteils 2 gegenüber dem in der Zeichnung oberen endständigen Implantatteil 1 durch den Anschlag seiner Hülse 5" am mittleren Implantatteil 3 bestimmt ist. Die Hülse des mit dem mittleren Implantatteil 3 gewindemäßig verbundenen, also oberen endständigen Implantatteils 1 trägt das Gewinde 4 auf ihrer äußeren Umfangsfläche. In dieses Gewinde 4 greift das die Hülse 5' radial an ihrer Außenseite umgebende mittlere Implantatteil 3 mit einem Muttergewinde 4'. Die Hülse 5' des mit dem mittleren Implantatteil 3 gewindemäßig verbundenen endständigen, also in der Zeichnung oberen Implantatteils 1 ist außer vom mittleren Implantatteil 3 auch von der Hülse 5" des anderen endständigen Implantatteils 2 radial außen umfaßt. Zur gegenseitigen Verdrehungssicherung der beiden endständigen Implantatteile 1, 2 ist in der Hülsenwand des in der Zeichnung oberen endständigen Implantatteils 1 ein axial verlaufender Führungsschlitz 7 und an der Hülsenwand des anderen, in der Zeichnung unteren Implantatteils 2 ein in den Führungsschlitz 7 eingreifender Führungsvorsprung 8 vorgesehen. Der Führungsvorsprung 8 ist durch einen radial in die Hülsenwand eingesetzten Stift 8' gebildet. Der Führungsschlitz 7 bestimmt mit seiner Länge das Maß, um welches die beiden endständigen Implantatteile 1, 2 gegeneinander axial verschoben werden können. Weiter besitzt die Hülse 5" des unteren endständigen Implantatteils 2 im Überlappungsbereich mit der Hülse 5' des oberen endständigen Implantatteils 1 mindestens eine radiale Gewindebohrung 9, in der eine nur in Fig. 1 und 2 dargestellte, gegen die Hülse 5' des oberen endständigen Implantarteils 1 verspannbare Klemmschraube 10 geführt ist, so daß bei angezogener Klemmschraube 10 die beiden endständigen Implantatteile 1, 2 axial nicht auseinandergezogen werden können. Weiter ist das obere endständige Implantatteil 1 am vom mittleren Implantatteil 3 abgewandten Ende mit einer über den Gewindedurchmesser radial nach außen vorstehenden ringförmigen Stirnplatte 11 versehen, an deren Stirnfläche zum Eindringen in den angrenzenden Wirbel bestimmte Schneiden oder Spitzen 12' vorgesehen sind. Entsprechende Schneiden bzw. Spitzen 12" befinden sich am axial gegenüberliegenden stirnseitigen Hülsenrand des unteren endständigen Implantatteils 2. Das mittlere Implantatteil 3 ist an seiner äußeren Umfangsfläche mit über den Umfang verteilt angeordneten Aussparungen 13 versehen, die als Schlüsselöffnungen zum Einstecken eines zum Verdrehen des mittleren Implantatteiles 3 dienenden, selbst nicht dagestellten Schlüssels ausgebildet sind.

## Patentansprüche

1. Implantat zum Einsetzen zwischen Wirbelkörper als Platzhalter, bestehend aus zwei endständigen, zur Anlage an den angrenzenden Wirbeln bestimmten Implantatteilen (1, 2) und einem dazwischen befindlichen mittleren Implantatteil (3), das mit einem der endständigen Implantatteile durch ein Gewinde (4) verbunden ist, wobei die drei Implantatteile (1, 2, 3) und das Gewinde (4) in Längsrichtung der Wirbelsäule koaxial angeordnet sind und durch Verdrehen des mittleren Implantatteils (3) die Länge des Implantats insgesamt veränderbar ist, und wobei die endständigen Implantatteile (1, 2) rohrförmige Hülsen (5', 5") mit von Aussparungen (6) durchbrochenen Hülsenwänden bilden, **dadurch gekennzeichnet, daß** die beiden endständigen Implantatteile (1, 2) mit ihren Hülsen (5', 5") axial ineinander greifen und an den Hülsenwänden axial gegeneinander verschieblich geführt sowie gegen gegenseitiges Verdrehen um die Hülsenachse gesichert sind, daß das Gewinde (4) zwischen dem mittleren Implantatteil (3) und dem einzigen mit ihm gewindemäßig verbundenen endständigen Implantatteil (1) an dessen Hülse (5') vorgesehen ist und daß das im Gewinde (4) dieser Hülse (5') verdrehbare mittlere Implantatteil (3) als axialer Anschlag für das andere endständige Implantatteil (2) ausgebildet ist.

2. Implantat nach Anspruch 1, **dadurch gekennzeichnet, daß** die Hülse (5') des mit dem mittleren Implantatteil (3) gewindemäßig verbundenen endständigen Implantatteils (1) das Gewinde (4) auf seiner äußeren Umfangsfläche trägt und sowohl vom mittleren Implantatteil (3) als auch von der Hülse (5") des anderen endständigen Implantatteils (2) radial außen umfaßt ist.

3. Implantat nach Anspruch 1 oder 2, **dadurch gekennzeichnet, daß** zur gegenseitigen Verdrehungssicherung der beiden endständigen Implantatteile (1, 2) in der Hülsenwand des einen Implantateils (1) ein axial verlaufender Führungsschlitz (7) und an der Hülsenwand des anderen Implantatteils (2) ein in den Führungsschlitz (7) eingreifender Führungsvorsprung (8) vorgesehen ist.

4. Implantat nach einem der Ansprüche 1 bis 3, **dadurch gekennzeichnet, daß** die Hülse (5") eines der endständigen Implantatteile (1, 2) im Überlappungsbereich der Hülsen (5', 5") beider endständiger Implantatteile (1, 2) mindestens eine radiale Gewindebohrung (9) aufweist, in der eine gegen die Hülse (5') des anderen endständigen Implantatteils (1) verspannbare Klemmschraube (10) geführt ist.

5. Implantat nach einem der Ansprüche 1 bis 4, **dadurch gekennzeichnet, daß** eines der beiden endständigen Implantatteile (1, 2) am vom mittleren Implantatteil (3) abgewandten Ende mit einer ringförmigen Stirnplatte (11) und diese Stirnplatte (11) an ihrer Stirnfläche mit zum Eindringen in den angrenzenden Wirbel bestimmten Schneiden oder Spitzen (12', 12") versehen ist.

6. Implantat nach einem der Ansprüche 1 bis 5, **dadurch gekennzeichnet, daß** das mittlere Implantatteil (3) an seiner äußeren Umfangsfläche über den Umfang verteilt angeordnete Ausparungen (13) aufweist, die als Schlüsselöffnungen zum Einstecken eines zum Verdrehen des mittleren Implantatteils (3) dienenden Schlüssels ausgebildet sind.

## Claims

1. Implant for insertion between the bodies of vertebrae as a spacer, consisting of two terminal implant elements (1, 2) which will be in contact with the adjacent vertebrae and situated therebetween a central implant element (3) which is joined to one of the terminal implant elements by means of a thread (4), the three implant elements (1, 2, 3) and the thread (4) being arranged coaxially in the longitudinal direction of the vertebral column and the length of the implant being modifiable overall by twisting the central implant element (3), and the terminal implant elements (1, 2) forming tubular sleeves (5', 5") with sleeve walls incorporating recesses (6), **characterised in that** the two terminal implant elements (1, 2) engage axially in one another by their sleeves (5', 5") and are set so as to be axially displaceable against one another at the sleeve walls and are locked against twisting relative to one another about the sleeve's axis, that the thread (4) between the central implant element (3) and the single terminal implant element (1) threaded thereonto is provided on the sleeve (5') of said implant element (1), and that the central implant element (3) adapted to be twisted in the thread (4) of said sleeve (5') is constructed in the form of an axial limit stop for the other terminal implant element (2).

2. Implant according to claim 1, **characterised in that** the sleeve (5') of the terminal implant element (1) threaded onto the central implant element (3) has the thread (4) on its outer circumferential surface and is radially encompassed on the outside both by the central implant element (3) and by the sleeve (5")of the other terminal implant element (2).

3. Implant according to Claim 1 or 2, **characterised in that** in order to mutually lock the two terminal implant elements (1, 2) against torsion, an axially running guide slot (7) is provided in the sleeve wall of one implant element (1), and a guide collar (8) which engages in the guide slot (7) is provided on the sleeve wall of the other implant element (2).

4. Implant according to any of claims 1 to 3, **characterised in that** in the overlapping region of the sleeves (5', 5") of the two terminal implant elements (1, 2), the sleeve (5") of one of the terminal implant elements (1, 2) incorporates at least one radial threaded bore (9) in which is set an adjusting screw (10) which is adapted to be tensioned against the sleeve (5') of the other terminal implant element (1).

5. Implant according to any of claims 1 to 4, **characterised in that** one of the two terminal implant elements (1, 2) is equipped with an annular face plate (11) on the end furthest from the central implant element (3), and this face plate (11) is equipped on its end surface with bezels or tips (12, 12") for penetrating into the adjoining vertebra.

6. Implant according to any of claims 1 to 5, **characterised in that** on its external circumferential surface the central implant element (3) incorporates recesses (13) arranged distributed around the circumference, which are constructed in the form of keyhole apertures for the insertion of a key used for twisting the central implant element (3).

## Revendications

1. Implant à insérer entre des vertèbres en tant qu'élément pour combler un espace vide, comprenant deux éléments d'extrémité (1, 2) destinés à venir en appui sur les vertèbres voisines et un élément médian (3) qui est lié par un filetage (4) à l'un des éléments d'extrémité, les trois éléments (1, 2, 3) et le filetage (4) étant disposés coaxialement dans la direction longitudinale de la colonne vertébrale et la longueur de l'implant pouvant être globalement modifiée en tournant l'élément médian (3), les éléments d'extrémité (1, 2) se présentant sous la forme de manchons tubulaires (5', 5"), avec des parois percées d'ouvertures (6), **caractérisé en ce que** les deux éléments d'extrémité (1, 2) rentrent axialement l'un dans l'autre avec leurs manchons (5', 5"), sont guidés avec possibilité de coulissement dans la direction axiale l'un par rapport à l'autre au niveau de leurs parois et sont freinés en rotation autour de l'axe de manchon, **en ce que** le filetage (4) entre l'élément médian (3) et l'élément d'extrémité (1) unique lié à celui-ci par le filetage est aménagé dans le manchon (5') dudit élément d'extrémité et **en ce que** l'élément médian (3) qui peut tourner dans le filetage (4) dudit manchon est conformé en butée axiale pour l'autre élément d'extrémité (2).

2. Implant selon la revendication 1, **caractérisé en ce que** le manchon (5') de l'élément d'extrémité (1) lié par filetage à l'élément médian (3) porte le filetage (4) sur sa paroi extérieure et est entouré extérieurement dans la direction radiale à la fois par l'élément médian (3) et par le manchon (5'') de l'autre élément d'extrémité (2).

3. Implant selon la revendication 1 ou 2, **caractérisé en ce que**, pour immobiliser en rotation l'un par rapport à l'autre les deux éléments d'extrémité (1, 2), il est prévu dans la paroi de manchon de l'un des éléments d'extrémité (1), une fente de guidage axiale (7) et, sur la paroi de manchon de l'autre élément d'extrémité (2), une saillie de guidage (8) qui s'engage dans la fente de guidage (7).

4. Implant selon une des revendications 1 à 3, **caractérisé en ce que** le manchon de l'un des éléments d'extrémité (1, 2), dans la zone de recouvrement des manchons (5', 5") desdits deux éléments d'extrémité (1, 2), présente au moins un trou fileté (9) radial, dans lequel pénètre une vis de blocage (10), qui peut être serrée contre le manchon (5') de l'autre élément d'extrémité (1).

5. Implant selon une des revendications 1 à 4, **caractérisé en ce que** l'un des deux éléments d'extrémité (1, 2), à son bout éloigné de l'élément médian (3), est muni d'une plaque frontale (11) annulaire et **en ce que** ladite plaque frontale (11) est pourvue sur sa face frontale d'arêtes ou de pointes (12', 12") destinées à pénétrer dans la vertèbre voisine.

6. Implant selon une des revendications 1 à 5, **caractérisé en ce que** l'élément médian (3) présente sur sa surface périphérique extérieure des ouvertures (13) uniformément réparties, qui sont conformées en empreintes de clé pour l'insertion d'une clé aux fins de tourner l'élément médian (3).
